(19) Europäisches Patentamt
European Patent Office
Office européen des brevets

(11) Publication number: **0 237 108 B1**

(12) **EUROPEAN PATENT SPECIFICATION**

(45) Date of publication of the patent specification: **26.09.90**

(51) Int. Cl.[5]: **C07C 45/34**, C07C 47/048, C07C 47/07, C07C 45/35, C07C 47/02, C07C 47/06, C07C 47/04

(21) Application number: **87200328.0**

(22) Date of filing: **25.02.87**

(54) **Process for the preparation of carbonyl compounds.**

(30) Priority: **06.03.86 GB 8605534**

(43) Date of publication of application: **16.09.87 Bulletin 87/38**

(45) Publication of the grant of the patent: **26.09.90 Bulletin 90/39**

(84) Designated Contracting States: **AT BE DE ES FR GB IT NL**

(56) References cited:
**DE-A- 1 135 441**
**DE-A- 1 643 688**
**DE-A- 1 956 606**
**FR-A- 1 524 016**
**FR-A- 2 071 424**
**GB-A- 1 043 606**
**US-A- 3 365 499**

(73) Proprietor: **SHELL INTERNATIONALE RESEARCH MAATSCHAPPIJ B.V., Carel van Bylandtlaan 30, NL-2596 HR Den Haag(NL)**

(72) Inventor: **Drent, Eit, Badhuisweg 3, NL-1031 CM Amsterdam(NL)**

(74) Representative: **Aalbers, Onno et al, P.O. Box 302, NL-2501 CH The Hague(NL)**

EP 0 237 108 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of a carbonyl compound by oxidation of an olefinically unsaturated compound in the presence of oxygen and water and a catalyst of a compound of a noble metal of Group 8 of the Periodic Table of the Elements, a salt of a metal showing at least two valence states as a redox system, and a quinone.

According to US patent specification 3 076 032 an olefinic carbon atom in the olefinically unsaturated compound is oxidized by this known process to a carbonyl group. This known process may be supported by using an organic solvent which is preferably miscible with water, for example acetic acid, methyl ethyl ketone or other ketones, mono- or polyhydric alcohols, acyclic ethers or dimethylformamide. However, applicant have observed that using the dimethyl ether of diethyleneglycol and dimethylformamide as solvents in this known process gives an extremely low conversion of the starting olefinically unsaturated compound.

West German patent application publication number DE-A 1 643 688 discloses a process for oxidizing olefins which comprises contacting the olefin, a Group 8 metal, a quinone, water and an organic solvent. The organic solvent may be N-methylpyrrolidone. During the process the quinone is reduced to hydroquinone. This hxdroquinone is recovered, oxidized back to quinone and recycled.

United States patent specification number US 3 365 499 discloses a process for the preparation of carbonyl compounds which comprises contacting an olefin with water, a platinum group metal salt, a quinone and an aprotic organic solvent. The examples illustrate the use of dimethylformamide as the aprotic organic solvent. A comparative example illustrates a process in which copper chloride and oxygen are used instead of a quinone.

West German patent application publication number DE-A 1 956 606 discloses a process for the preparation of carbonyl compounds which comprises contacting an olefin with oxygen, water, a palladium salt, a copper compound, an iron compound and an N, N-dialkylcarboxamide such an N-methylpyrrolidone.

French patent specification number 2 071 424 discloses a process for the preparation of carbonyl compounds which comprises contacting an olefin with oxygen, water, a palladium salt, a copper salt and a lactam such as N-methylpyrrolidone.

It has now surprisingly been found that using a special type of solvents allows a high conversion of the starting olefinically unsaturated compound, with a very high selectivity to carbonyl compounds. The selectivity to a certain compound, expressed in a percentage, is defined herein as 100 x a/b, in which "a" is the amount of starting olefinically unsaturated compound that has been converted into that certain compound and "b" is the total amount of starting olefinically unsaturated compound that has been converted.

Accordingly, the invention provides a process for the preparation of a carbonyl compound which comprises oxidizing an olefinically unsaturated compound in the presence of oxygen and water and a catalyst comprising a noble metal of Group 8 of the Periodic Table of the Elements or a compound thereof, a salt of a metal showing at least two valence states as a redox system, and a quinone, which process is carried out in the presence of a solvent comprising a carbamoyl group

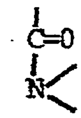

in which the nitrogen atom is bound to three carbon atoms and in which the carbon atom is bound to another carbon atom.

The solvents which are preferably used in the process according to the present invention are N,N-dimethylacetamide and N-methyl-2-pyrrolidone. Other examples of suitable solvents are N-methyl-N-ethylacetamide, N,N-diethylacetamide, N,N-dipropylacetamide, N-butyl-N-phenylacetamide, N,N-dimethylpropanamide, N,N-diethylpropanamide, N-methyl-N-ethylpropanamide, N-ethyl-2-pyrrolidone and N-methyl- and N-ethyl-caprolactam. Mixtures of two or more of these solvents may be used.

The presence of solvents other than those having a carbamoyl group in which the nitrogen atom is bound to three carbon atoms is not excluded, provided they are present in an amount of less than 50% by weight, calculated on the sum of all solvents in question. Examples of such solvents are alkanols, such as methanol, ethanol, propanol, 2-propanol ethylene glycol and 1,4-butanediol; ethers, such as methyl ethyl ether, diethyl ether, dipropyl ether, tetrahydrofuran, dimethyl ether of diethylene glycol (also referred to as "diglyme"), methyl tert.-butyl ether, dichloroethyl ether, ethyl phenyl ether, diethylene glycol diethyl ether and 1,4-dioxane. Other examples of suitable such solvents are halogenated hydrocarbons, such as chloroform, chlorobenzene, carbon tetrachloride and perfluoroalkanes; esters, such as the methyl and ethyl esters of formic acid, acetic acid, adipic acid, succinic acid, propionic acid, oxalic acid and benzoic acid; sulphones such as dimethyl sulphone, methyl butyl sulphone and tetrahydrothiophene 1,1-dioxide (also referred to as "sulfolane"); aromatic hydrocarbons such as benzene, toluene and the three xylenes; cycloalkanes such as cyclohexane and cyclooctane, and nitro compounds, such as nitrobenzene.

The noble metals which are used in the process according to the present invention are platinum, rhodium, ruthenium, palladium , iridium and/or osmium. These metals may be used in metallic form or as compounds. Mixtures of compounds of the same or different such noble metals or mixtures of such noble metals in metallic form may be used. The noble metals may be used as finely divided metals, not supported on a carrier, or supported on a carrier, for example on activated carbon, pumice or graphite. The present process is preferably carried out in the presence of palladium and/or a compound of palladium. Very good results have been obtained with

compounds of palladium. Among the palladium compounds palladium(II) salts of alkanoic acids having not more than 12 carbon atoms per molecule are preferred. Palladium(II) acetate is particularly preferred. Other examples of suitable compounds of Group 8 noble metals are salts, such as nitrates, sulphates and halides (fluorides, chlorides, bromides and iodides).

Further examples of suitable palladium compounds are palladium complexes such as bis(2,4-pentanedionato)palladium, bis(picolinato)palladium, tetrakis(triphenylphosphine)palladium, tris(dibenzylidene-acetone)dipalladium, bis(triphenylphosphine)-(1,4-benzoquinone)palladium, tetrakisacetonitrilepalladium tetrafluoroborate, bis(tri-o-tolylphosphine)palladium acetate, bis(triphenylphosphine)palladium sulphate, palladium olefin complexes, for instance di-μ-chloro-dichlorobis(ethylene)dipalladium ($[Pd.C_2H_4.Cl_2]_2$), and di-μ-chlorodichlorobis(propylene)dipalladium ($[Pd.C_3H_6.Cl_2]_2$), and palladiumhydride complexes. Palladium may be used in complex combination with phosphites, such as triphenyl phosphite or tributyl phosphite. Mixtures of two or more of such palladium compounds may be used.

The redox system which is used in the process according to the present invention is a salt of a metal showing two or more valence states under the conditions at which the process is carried out. Redox systems are well known in the art. Preferred redox systems comprise a compound of copper, iron, vanadium, cobalt and/or manganese. These five metals are preferably used in the form of salts, such as halides (fluorides, chlorides, bromides or iodides), nitrates, sulphates, carboxylates (preferably those carboxylates having not more than 12 carbon atoms per molecule), perchlorates and sulphonates, for instance benzenesulphonates and p-tosylates. Among the metal compounds cupric compounds are preferred, particularly cupric chloride. Further examples of suitable redox systems are cobalt(II) complexes of organic ligands, cupric acetate, cupric butyrate, cupric bromide, ferrous propionate, ferric acetate, ferric chloride, ferric bromide cobalt(II) acetate, cupric perchlorate, cupric tosylate and vanadyl sulphate ($VOSO_4$). Other examples of redox systems comprise compounds of mercury, cerium, thallium, tin, lead, titanium, chromium, molybdenum, uranium and nickel.

The quinone applied in the process according to the present invention may be an ortho-quinone or a para-quinone and may be, for example, a benzoquinone, a naphthoquinone, an anthraquinone or a chrysenequinone. Preference is given to optionally substituted benzoquinones, particularly to p-benzoquinones. According to a preferred embodiment of the present invention methyl-substituted p-benzoquinones are present, very high yields of carbonyl compounds being obtained. Examples of such quinones are 2-methyl-p-benzoquinone, 2,3-dimethyl-p-benzoquinone, 2,5-dimethyl-p-benzoquinone, 2,6-dimethyl-p-benzoquinone and 2,3,5-trimethyl-p-benzoquinone. Very good results have been obtained with 2,3,5,6-tetramethyl-p-benzoquinone.

The process according to the present invention can also suitably be carried out in the presence of halogen-substituted p-benzoquinones, one or more fluorine, chlorine, bromine and/or iodine atoms being attached to the aromatic nucleus of the p-benzoquinone. Examples of such quinones are 2-iodo-, 2-bromo-, 2-chloro- and 2-fluoro-p-benzoquinone, 2,6-diiodo-, 2,6-dibromo-, 2,6-dichloro- and 2,6-difluoro-p-benzoquinone, 2,3,6-triiodo-, 2,3,6-tribromo-, 2,3,6-trichloro- and 2,3,6-trifluoro-p-benzoquinone, 2,3,5,6-tetraiodo-, 2,3,5,6-tetrabromo-, 2,3,5,6-tetrachloro- and 2,3,5,6-tetrafluoro-p-benzoquinone. Further examples of suitable quinones are 9,10-anthraquinone, 1,4-naphthoquinone and 5,6-chrysenequinone. The quinone may have been rendered water-soluble by substitution with sulphonic acid and/or carboxylic groups. Examples of such quinones are potassium 1,2-naphtho quinone-4-sulphonate, 1,2-naphthoquinone-4-sulphonic acid and 1,4-naphthoquinone-2-carboxylic acid.

The following molar ratios are not critical and may vary within wide ranges:-

a) compound of a noble metal of Group 8 to olefinically unsaturated compound,

b) redox agent to compound of a noble metal of Group 8, and

c) quinone to compound of a noble metal of Group 8.

It is a feature of the present process that even when the molar ratio sub(a) is low, high conversions of olefinically unsaturated compound are obtained. This molar ratio is preferably in the range of from $10^{-2}$to $10^{-6}$, but the use of molar ratios higher than $10^{-2}$ or lower than $10^{-6}$ is not excluded. The molar ratios sub (b) and (c) are preferably in the range of from 1 to 500, but the use of molar ratios below 1 and above 500 is not excluded.

The oxygen may be supplied as pure oxygen, in air enriched with oxygen, in air or diluted with an inert gas such as argon, carbon dioxide or methane. The process may be carried out using a partial pressure of oxygen which is not critical and may vary within wide ranges, suitably in the range of from 0.1 to 100 bar, preferably from 0.1 to 50 bar and more preferably from 1 to 20 bar. The stoichiometric molar ratio olefinically unsaturated compound to oxygen is 2 : 1; for safety reasons, a deficiency of oxygen is suitably present, for example in the range of from 2.5 : 1 to 4 : 1 and the oxygen is suitably supplied in portions to the reaction mixture.

The process according to the invention may be carried out at a temperature which is not critical and may vary within wide ranges, suitably in the range of from 20 °C to 200 °C.

The olefinically unsaturated compound has a number of carbon atoms which is not critical and may vary within wide ranges, and has at least one hydrogen atom bound to the C = C group. Preferably, the olefinically unsaturated compound is an optionally substituted alkene having in the range of from 2 to 20 carbon atoms per molecule or an optionally substituted cycloalkene having in the range of from 3 to 20 carbon atoms per molecule. The olefinically unsaturated compound is more preferably an alkene having in the range of from 4 to 10 carbon atoms per molecule. Mixtures of olefinically unsaturated compounds may be used. Very good results have been obtained with 1-butene, 2-butene and with mixtures thereof, yielding methyl ethyl ketone with very high selectivity, only very small quantities of butanal being formed. Other examples of suitable olefinically unsaturated compounds are ethylene (yielding acetaldehyde), propene (yielding acetone), 1-pentene (yielding 2-pentanone), 2-pentene (yielding a mixture of 2-pentanone and 3-pentanone), 1-hexene (yielding 2-hexanone), 2-hexene (yielding a mixture of 2-hexanone and 3-hexanone), 3-hexene (yielding 3-hexanone), 1-octene (yielding 2-octanone), 2-dodecene (yielding a mixture of 2-dodecanone and 3-dodecanone), 2-methyl-1-butene (yielding 2-methylbutanal), 2-methyl-2-butene (yielding 3-methyl-2-butanone), styrene (yielding acetophenone), cyclopentene (yielding cyclopentanone), cyclohexene (yielding cyclohexanone) and cyclooctene (yielding cyclooctanone). The olefinically unsaturated compound may contain substituents, such as carbonyl groups, alkoxy groups (preferably those containing less than 5 carbon atoms) and nitro groups. The olefinically unsaturated compound may contain two or more carbon carbon double bonds per molecule, for example in 1,4-pentadiene (yielding 2,4-pentanedione) and 1,4-cyclooctadiene (yielding a mixture of 1,2-, 1,3-, 1,4- and 1,5-cyclooctanedione).

The process according to the invention can be carried out batchwise, semi-continuously or continuously. The reaction time varies in relation to the temperature used and is usually between 0.5 and 20 hours.

The following Examples further illustrate the invention. The octanones formed in Examples 1-8 had a content of 2-octanone of more than 95%.

Example 1

A 300 ml magnetically stirred Hastelloy C autoclave ("Hastelloy" is a trade name) was charged with Pd(II) acetate (0.1 mmol), N-methyl-2-pyrrolidone (45 ml), water (5 ml), 1-octene (10 ml, 64 mmol), cupric chloride (10 mmol) and 2,3,5,6-tetramethylbenzoquinone (20 mmol). After pressurising with air at a temperature of 20 °C until a pressure of 30 bar was obtained the autoclave was kept for 1.5 hour at a temperature of 85 °C. At the end of this period the contents of the autoclave were analyzed by means of gas-liquid chromatography.

The conversion of 1-octene was 51% with a selectivity to octanones of 97% and to octanal of 3%.

Example 2

Example 1 was repeated except that not 5 ml but 0.5 ml of water was present.

The conversion of 1-octene was 39% with a selectivity to octanones of 96% and to octanal of 3%.

Example 3

Example 1 was repeated except that not 2,3,5,6-tetramethylbenzoquinone (20 mmol) but 2,3,5,6-tetrachlorobenzoquinone (20 mmol) was present.

The conversion of 1-octene was 37.3% with a selectivity to octanones of 99% and to octanal of 1%.

Example 4

Example 1 was repeated except that cupric chloride (10 mmol) was replaced with ferric chloride (10 mmol).

The conversion of 1-octene was 47% with a selectivity to octanones of 96% and to octanal of 3.5%.

Example 5

Example 1 was repeated except that cupric chloride (10 mmol) was replaced with vanadyl sulphate ($VOSO_4$, 10 mmol).

The conversion of 1-octene was 18% with a selectivity to octanones of 97% and to octanal of 3%.

Example 6

Example 1 was repeated except that cupric chloride (10 mmol) was replaced with cupric perchlorate (10 mmol).

The conversion of 1-octene was 16% with a selectivity to octanones of 97% and to octanal of 3%.

Example 7

Example 1 was repeated except that cupric chloride (10 mmol) was replaced with cupric tosylate (10 mmol).

The conversion of 1-octene was 12.5% with a selectivity to octanones of 98% and to octanal of 2%.

Example 8

Example 1 was repeated except that N-methyl-2-pyrrolidone (45 ml) was replaced with N,N-dimethylacetamide (45 ml).

The conversion of 1-octene was 50% with a selectivity to octanones of 97.5%.

Example 9

Example 1 was repeated except that 1-octene (10 ml) was replaced with 10 ml of an equimolar mixture of 1-butene and 2-butene.

The conversion of the butenes was 30% with a selectivity to methyl ethyl ketone of 97% and to butanal of 3%.

Example 10

An experiment was carried out as described hereinbefore, using palladium(II) acetate (0.1 mmol), N-methyl-2-pyrrolidone (45 ml), water (5 ml), 1-butene (10 ml), cupric chloride (10 mmol), 2,3,5,6-tetrachloro-p-benzoquinone (20 mmol) and air (15 bar).

The conversion of 1-butene after 1.5 h at 85 °C was 50% with a selectivity to methyl ethyl ketone of 98%.

Comparative Example A

Example 1 was repeated except that N-methyl-2-pyrrolidone (45 ml) was replaced with N,N-dimethylformamide (45 ml).

The conversion of 1-octene was only 3%.

Comparative Example B

Example 1 was repeated except that N-methyl-2-pyrrolidone (45 ml) was replaced with N,N-dimethylformamide (45 ml) and that only 1 ml of water was present.

The conversion of 1-octene was only 6.5%.

Comparative Example C

Example 1 was repeated except that N-methyl-2-pyrrolidone (45 ml) was replaced with tetrahydrothiophene 1,1-dioxide (45 ml).

The conversion of 1-octene was less than 1%.

Comparative Example D

Example 1 was repeated except that N-methyl-2-pyrrolidone (45 ml) was replaced with the dimethyl ether of diethyleneglycol.

The conversion of 1-octene was less than 1%.

Comparative Example E

Example 10 was repeated except that 2,3,5,6-tetrachloro-p-benzoquinone was not present.

The conversion of 1-butene after 5 h at 85 °C was only 15%.

Comparative Example F

Example 1 was repeated except that cupric chloride was not present.

The conversion of 1-octene after 1.5 h at 85 °C was less than 2%.

## Claims

1. A process for the preparation of a carbonyl compound which comprises oxidizing an olefinically unsaturated compound in the presence of oxygen and water and a catalyst comprising a noble metal of Group 8 of the Periodic Table of the Elements or a compound thereof, a salt of a metal showing at least two valence states as a redox system, and a quinone, which process is carried out in the presence of a solvent containing a carbamoyl group

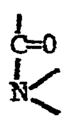

in which the nitrogen atom is bound to three carbon atoms and in which the carbon atom is bound to another carbon atom.

2. A process as claimed in claim 1 in which the solvent is N-methyl-2-pyrrolidone.

3. A process as claimed in claim 1 in which the solvent is N,N-dimethylacetamide.

4. A process as claimed in any one of the preceding claims which is carried out in the presence of palladium and/or a compound of palladium.

5. A process as claimed in claim 4 in which said palladium compound is a palladium(II) salt of an alkanoic acid having not more than 12 carbon atoms per molecule.

6. A process as claimed in any one of the preceding claims in which said redox system comprises a compound of copper, iron, vanadium, cobalt and/or manganese.

7. A process as claimed in claim 6 in which said redox agent comprises a cupric compound.

8. A process as claimed in any one of the preceding claims in which the quinone is an optionally substituted benzoquinone.

9. A process as claimed in claim 8 in which the quinone is 2,3,5,6-tetramethyl-p-benzoquinone.

10. A process as claimed in any one of the preceding claims in which a molar ratio redox agent to compound of a noble metal of Group 8 in the range of from 1 to 500 is used.

11. A process as claimed in any one of the preceding claims in which a molar ratio quinone to compound of a noble metal of Group 8 in the range of from 1 to 500 is used.

12. A process as claimed in any one of the preceding claims which is carried out at a partial pressure of oxygen in the range of from 0.1 to 50 bar.

13. A process as claimed in any one of the preceding claims in which a temperature is maintained in the range of from 20 °C to 200 °C.

14. A process as claimed in any one of the preceding claims in which the olefinically unsaturated compound is an optionally substituted alkene having in the range of from 2 to 20 carbon atoms per molecule or an optionally substituted cycloalkene having in the range of from 3 to 20 carbon atoms per molecule.

## Revendications

1. Un procédé pour la préparation d'un composé carbonylé qui comprend l'oxydation d'un composé oléfiniquement insaturé en présence d'oxygène et d'eau et d'un catalyseur comprenant un métal noble du groupe 8 du tableau périodique des éléments ou un composé d'un tel métal, un sel d'un métal présentant au moins deux états de valence sous la forme d'un système redox et une quinone, procédé qui est mis en œuvre en présence d'un solvant comprenant un groupe carbamoyle

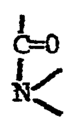

dans lequel l'atome d'azote est lié à trois atomes de carbone et l'atome de carbone est lié à un autre atome de carbone.

2. Un procédé selon la revendication 1, dans lequel le solvant est la N-méthyl-2-pyrrolidone.

3. Un procédé selon la revendication 1, dans lequel le solvant est le N,N-diméthylacétamide.

4. Un procédé selon l'une quelconque des revendications précédentes, qui est mis en œuvre en présence de palladium et/ou d'un composé du palladium.

5. Un procédé selon la revendication 4, dans lequel le composé du palladium est un sel de palladium(II) d'un acide alcanoïque n'ayant pas plus de 12 atomes de carbone par molécule.

6. Un procédé selon l'une quelconque des revendications précédentes, dans lequel le système redox comprend un composé du cuivre, du fer, du vanadium, du cobalt et/ou du manganèse.

7. Un procédé selon la revendication 6, dans lequel l'agent redox comprend un composé cuivrique.

8. Un procédé selon l'une quelconque des revendications précédentes, dans lequel la quinone est une benzoquinone éventuellement substituée.

9. Un procédé selon la revendication 8, dans lequel la quinone est la 2,3,5,6-tétraméthyl-p-benzoquinone.

10. Un procédé selon l'une quelconque des revendications précédentes, dans lequel on utilise un rapport molaire de l'agent redox au composé d'un métal noble du groupe 8 compris entre 1 et 500.

11. Un procédé selon l'une quelconque des revendications précédentes, dans lequel on utilise un rapport molaire de la quinone au composé d'un métal noble du groupe 8 compris entre 1 et 500.

12. Un procédé selon l'une quelconque des revendications précédentes, qui est mis en œuvre sous une pression partielle d'oxygène comprise entre 0,1 et 50 bars.

13. Un procédé selon l'une quelconque des revendications précédentes, dans lequel on maintient une température comprise entre 20°C et 200°C.

14. Un procédé selon l'une quelconque des revendications précédentes, dans lequel le composé oléfiniquement insaturé est un alcène éventuellement substitué ayant de 2 à 20 atomes de carbone par molécule ou un cycloalcène éventuellement substitué ayant de 3 à 20 atomes de carbone par molécule.

## Patentansprüche

1. Verfahren zur Herstellung einer Carbonylverbindung, das ein Oxidieren einer olefinisch ungesättigten Verbindung in Gegenwart von Sauerstoff und Wasser und eines Katalysators, der ein Edelmetall aus der Gruppe 8 des Periodensystems der Elemente oder eine Verbindung hievon umfaßt, eines Salzes eines wenigstens zwei Valenzzustände aufweisenden Metalles als ein Redox-System und eines Chinons umfaßt, welches Verfahren in Gegenwart eines Lösungsmittels ausgeführt wird, das eine Carbamoylgruppe

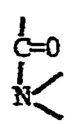

enthält, worin das Stickstoffatom an drei Kohlenstoffatome gebunden ist und worin das Kohlenstoffatom an ein weiteres Kohlenstoffatom gebunden ist.

2. Verfahren nach Anspruch 1, worin das Lösungsmittel N-Methyl-2-pyrrolidon ist.

3. Verfahren nach Anspruch 1, worin das Lösungsmittel N,N-Dimethylacetamid ist.

4. Verfahren nach einem der vorstehenden Ansprüche, das in Gegenwart von Palladium und/oder einer Palladiumverbindung ausgeführt wird.

5. Verfahren nach Anspruch 4, worin die Palladiumverbindung ein Palladium(II)-Salz einer Alkansäure mit nicht mehr als 12 Kohlenstoffatomen pro Molekül ist.

6. Verfahren nach einem der vorstehenden Ansprüche, worin das Redox-System eine Verbindung von Kupfer, Eisen, Vanadium, Kobalt und/oder Mangan umfaßt.

7. Verfahren nach Anspruch 6, worin das Redox-Mittel eine Kupfer(II)-Verbindung umfaßt.

8. Verfahren nach einem der vorstehenden Ansprüche, worin das Chinon ein gegebenenfalls substituiertes Benzochinon ist.

9. Verfahren nach Anspruch 8, worin das Chinon 2,3,5,6-Tetramethyl-p-benzochinon ist.

10. Verfahren nach einem der vorstehenden Ansprüche, worin ein Molverhältnis von Redox-Mittel zu Verbindung eines Edelmetalles der Gruppe 8 im Bereich von 1 bis 500 angewendet wird.

11. Verfahren nach einem der vorstehenden Ansprüche, worin ein Mol-Verhältnis von Chinon zu Verbindung eines Edelmetalls der Gruppe 8 im Bereich von 1 bis 500 angewendet wird.

12. Verfahren nach einem der vorstehenden Ansprüche, das bei einem Sauerstoffpartialdruck im Bereich von 0,1 bis 50 bar ausgeführt wird.

13. Verfahren nach einem der vorstehenden Ansprüche, worin eine Temperatur im Bereich von 20°C bis 200°C aufrecht erhalten wird.

14. Verfahren nach einem der vorstehenden Ansprüche, worin die olefinisch ungesättigte Verbindung ein gegebenenfalls substituiertes Alken mit 2 bis 20 Kohlenstoffatomen pro Molekül oder ein gegebenenfalls substituiertes Cycloalken mit 3 bis 20 Kohlenstoffatomen pro Molekül ist.